# EUROPEAN PATENT APPLICATION

(11) **EP 4 834 266 A1**
(43) Date of publication of application: **04.11.2026**
(21) Application number: 25173511.4
(22) Date of filing: 30.04.2025
(51) Int. Cl.: A61B 34/20, A61B 90/00, A61B 17/00

(54) **INDUCTIVELY-POWERED OPTICALLY TRACKABLE ELEMENT**

(71) Applicant: Stryker European Operations Limited, Carrigtwohill, Co. Cork T45 HX08 (IE)
(72) Inventor: RIEGELSBERGER, Fabian, 79211 Denzlingen (DE); KOEPFF, Marvin, 79227 Schallstadt (DE); UMBDENSTOCK, Emeric, 00185 Roma (IT); HERRMANN, Florian, 77963 Schwanau (DE)
(74) Representative: Röthinger, Rainer

(57) **Abstract**

A trackable element for use in an optical tracking system is presented. The trackable element comprises an active element configured to emit light when being electrically powered. The trackable element further comprises an inductive energy receiver comprising a receiver coil configured to inductively receive energy. The inductive energy receiver is configured to electrically power the active element.

## Description

### Technical Field

The present disclosure generally relates to the field of optical tracking, for example in the context of surgical navigation. In particular, a trackable element for use in an optical tracking system, a system comprising the trackable element and a method of operating the system are presented.

### Background

In many surgical navigation scenarios, a position of a surgical instrument relative to a patient has to be detected and tracked. To this end, optically detectable trackers are attached to the patient and the instrument. Conventional optical trackers include optically detectable elements. The optically detectable elements may include passive elements (e.g., reflective spheres) or active elements (e.g., light emitting diodes, LEDs).

A tracker with active elements such as LEDs is typically battery-powered. Using a battery for powering the active elements results in a tracker having a considerable size and weight, compared to a tracker with optically detectable passive elements. Moreover, when optically detectable active elements are used, a battery state of the tracker has to be regularly checked to avoid trackers that stop operation during a surgery due to a draining battery. Using a battery also generates additional waste and costs.

### Summary

There is a need for an optically trackable active element that addresses at least some of the aforementioned or other problems.

According to a first aspect, a trackable element for use in an optical tracking system is provided. The trackable element comprises an active element configured to emit light when being electrically powered. The trackable element further comprises an inductive energy receiver comprising a first receiver coil configured to inductively receive energy. The inductive energy receiver is configured to electrically power the active element.

The trackable element may not comprise an energy storage for electrical energy, such as a capacitor or a battery. In such a configuration, the active element can substantially only be powered while the inductive energy receiver is inductively receiving energy. In alternative configurations, the trackable element may comprise a storage for electrical energy. Such an energy storage may comprise at least one of a capacitor (of sufficient capacity to power the active element) and a battery. The energy storage may allow continuous operation of the trackable element, e.g., in case of an only intermittent exposure of the receive coil to a field generated by an inductive energy emitter. As a result, for example, flickering of the active element and intermittent tracking of the trackable element may be avoided. The energy storage may be inductively (re-)chargeable, thus improving usability of the trackable element.

The trackable element may constitute an optical tracker or at least a part thereof. The active element may consist of or comprise an LED. The LED may be configured to emit light in the visible spectrum or in the infrared spectrum. The first receiver coil may consist of or comprise a sequence of windings configured to electrically power the active element. The first receiver coil may be coupled to the active element via an electrical circuit line, e.g., by one or more wires or one or more printed lines.

The trackable element may integrate two, three or more active elements in a predefined pattern. In some variants, the trackable element may include only a single active element. The trackable element including a single active element may be used as a particularly small and light-weight tracker compared to optically detectable trackers integrating multiple active elements. Moreover, power consumption is reduced.

Multiple individual trackable elements with each trackable element including a single active element may individually be attached to an object (e.g., a patient, a surgical instrument or a medical imaging device) so that a user can define, or select, an essentially rigid pattern of active elements. In such case, a virtual tracker may be defined based on the multiple individual trackable elements. The form of the virtual tracker may be easily adaptable due to the multiple trackable elements being repositionable relative to each other. Thus, the virtual tracker may be adaptable to any given surgical situation.

In some variants, the trackable element may comprise a flexible substrate configured to conform to a surface that is at least one of non-rigid and non-planar. For example, the flexible substrate may be configured to conform to a patient anatomy, e.g., the skin of patient and bodily features and curvatures. In other examples, the substrate may be configured to conform to a planar or a non-planar (e.g., curved) surface of a surgical instrument or medical imaging device. As a result, the trackable element may be attachable to a large variety of objects with possibly different surface forms and may thus be usable for a plethora of situations in navigated surgery.

In some variants, multiple trackable elements may be provided and the substrate of each trackable element may be shaped in a dedicated optically unambiguously identifiable geometry. Additionally or alternatively, each trackable element may comprise an identification element, i.e., another active or passive element, having a dedicated optically unambiguously identifiable geometry or color. The identification element may be or comprise an optically unambiguously identifiable pattern (e.g., comprising at least one of a triangle, a square, a polygon and a quick response, QR, code). In such or other implementations, the active elements of different trackable elements may have different radiation characteristics (e.g., in regard of different emission wavelengths, different temporal emission patterns, and so on). As a result, each trackable element may be unambiguously optically identifiable by a tracking camera.

In some variants, the trackable element may comprise a printed circuit board (PCB) configured to electrically couple the inductive energy receiver and the active element. The PCB may be any known PCB comprising conductive circuit lines for electrically coupling the inductive energy receiver and the active element. The PCB may be a flexible PCB that constitutes the flexible substrate. The flexible PCB may be any known flexible PCB, i.e., comprise the materials of any known flexible PCB.

At least the first receiver coil may be integrated into the PCB. As a result, a compact trackable element may be provided. Moreover, integrating components in the PCB, e.g., the first receiver coil, may protect the integrated components against damages and fluids. For example, the receiver coil may be protected from mechanical damage (e.g., due to touching of the trackable element during attaching or detaching of the trackable element to or from an object) or from blood or other bodily fluids. Further, the trackable element can easily be cleaned and disinfected and, thus, become reusable. In other variants, the trackable element may be configured as a disposable item.

In some variants, the inductive energy receiver may comprise a second receiver coil configured to inductively receive energy. The second receiver coil may be, or may be configured to be (e.g., via a flexible substrate portion), substantially orthogonally oriented relative to the first receiver coil. Additionally, the inductive energy receiver may comprise a third receiver coil that is configured to inductively receive energy. The third receiver coil may be, or may be configured to be (e.g., via a flexible substrate portion), substantially orthogonally oriented relative to the first and the second receiver coils. Since an efficiency of an inductive energy transfer can depend on the relative orientation between the inductive energy receiver and an inductive energy emitter, providing additional receiver coils (i.e., the second, the third or even further receiver coils in addition to the first receiver coil, with each receiver coil having a unique orientation) may improve the energy transfer efficiency.

In some variants, at least one of the second receiver coil, the third receiver coil and the at least one further receiver coil may be integrated into the PCB. Analogously to the first receiver coil, the further receiver coil(s) may thus be protected.

The trackable element may comprise an attachment element configured for adhesively attaching the trackable element to an object that is to be tracked. The attachment element may be an adhesive layer on the side of the (e.g., flexible) substrate facing towards the object to be tracked. The trackable element may be detachably attachable to the object that is to be tracked without having to physically alter the object and without the need of any further mechanisms (e.g., clamps or screws) for attaching or detaching the trackable element.

The trackable element may comprise (e.g., electric and/or electronic) components configured to set electric or visual parameters for a powering of the active element, e.g., to regulate a brightness of the active element. The components may comprise any known passive, active or electro-mechanic components such as resistors, capacitors, filters, diodes, integrated circuits and so on. For example, the trackable element may comprise at least one capacitor. The at least one capacitor may form an inductivity-capacitance- (LC-) resonator circuit with the first receiver coil.

Additionally or alternatively, the trackable element may comprise (e.g., electric or electronic) components configured to wireless transfer data (e.g., via a modulation of the emission characteristic of the active element). For example, data may be transferred to or received from or exchanged with a navigation system. The transferred data may be indicative of an operation status of the trackable element, e.g., of the activation of the active element. Operation of the active element of the trackable element may be controlled based on received data.

At least a part of the trackable element may be radiopaque (e.g., a soldering spot used to electrically connect the active element to an electrical circuit). Thus, the trackable element becomes detectable in medical image data generated using a radiation-based imaging technique, e.g., x-ray imaging. Based on corresponding positions of multiple tracking elements in the medical image data and in optical image data generated by a tracking camera, the medical image data can be registered with the optical image data (e.g., for navigation purposes).

The active element may be shaped in an optically unambiguous geometric form or the trackable element may comprise another active or passive optically detectable element shaped in an optically unambiguous geometric form, e.g., the identification element explained above. The optically unambiguous geometric form(s) may allow to unambiguously identify individual trackable elements. Unambiguously identifying individual trackable elements may facilitate navigation based on tracking of trackable elements the and/or error analysis, e.g., by easily identifying malfunctioning trackable elements.

In some variants, the trackable element may comprise at least one flexible portion and the first coil may comprise at least two separate winding portions connected by the at least one flexible portion. As a result, the at least one flexible portion may allow to place the at least two winding portions on top of each other, for example to stack the winding portions for achieving a higher number of (stacked) windings. Stacking more windings may result in an improved, i.e., higher, power reception, since the power reception generally correlates with the number of stacked windings. The flexible portion may thus increase the power reception as well as the adaptability und usability of the trackable element.

According to a second aspect, a system is provided. The system comprises at least one trackable element according to the first aspect. The system further comprises an inductive energy emitter configured to inductively transfer energy to the inductive energy receiver.

In some variants, the inductive energy emitter comprises one or more emitter coils. Each of the emitter coils may have a unique orientation. For example, the inductive energy emitter comprises a first emitter coil with a first orientation, a second emitter coils that is oriented substantially orthogonally to the first emitter coil and, optionally, a third emitter coil that is oriented substantially orthogonally to the first emitter coil and the second emitter coil. The one or more emitter coils may thus form corresponding counterparts to the one or more receiver coils of the trackable element described herein.

In some variants, the system may further comprise a tracking camera configured to track the at least one trackable element. The tracking camera may be a stereo camera. The tracking camera may have a sensitivity in at least one of the visible and the infrared spectrum. The tracking camera may be configured to generate tracking image data (e.g., at a certain framerate).

The inductive emitter may be configured to generate a magnetic field in synchronization with the framerate of the tracking camera. As a result, energy may be transferred to the tracking element in accordance with the framerate of the camera. In this manner, a pulsating energy transfer may be realized and the active element(s) may only become active when the tracking camera actually acquires image frames. Consequently, the power consumption as well as the average magnetic flux exposure of a patient may be reduced.

The inductive emitter may be integrated into the tracking camera. The inductive emitter may be configured to be coupled to any conventional tracking camera or may be integrated into the tracking camera in the manufacturing phase.

In some variants, the system may comprise multiple trackable elements according to the first aspect. In such variants, the inductive emitter may be configured to simultaneously transfer energy to the inductive receivers of at least two of the multiple trackable elements. For example, a single inductive emitter may be configured to simultaneously transfer energy to the inductive receivers of two, three, four or all of the provided trackable elements. In some variants, the system may comprise multiple inductive emitters. The inductive emitters may all be configured to generate a magnetic field in synchronization with the framerate of the tracking camera.

According to a third aspect, a method of operating a system such as the one of the second aspect is provided. The system comprises at least one trackable element according to the first aspect, an inductive energy emitter configured to inductively transfer energy to the inductive energy receiver of the at least one trackable element, and a tracking camera configured to track the at least one trackable element. The method comprises inductively transferring energy from the inductive emitter to the inductive energy receiver of the at least one trackable element. The method further comprises tracking, by the tracking camera, the active element of the at least one trackable element.

In some variants, at least two trackable elements may be attached to an object, and the method may further comprise tracking the at least two trackable elements attached to the object, and attaching at least one further trackable element based on (e.g., positioning instructions generated based on) tracking image data indicative of the at least two trackable elements attached to the object, for example to realize a target pattern of trackable elements on the object. The target pattern to be realized may correspond to a predefined virtual tracker.

In some variants, multiple trackable elements may be attached to the object in a user-selected pattern and the method may comprise determining the specific pattern based on tracking image data generated by the tracking camera. The method may further comprise determining at least one of a position and an orientation (e.g., a pose) of the object based on the determined user-selected pattern. Additionally, or in the alternative, the method may further comprise determining a malfunction of any of the multiple trackable elements based on the determined user-selected pattern (e.g., because of a change thereof). The user-selected pattern may be a previously unknown pattern generated by a user individually attaching the multiple trackable elements to the object to be tracked.

### Brief Description of the Drawings

Further details, advantages and aspects of the present disclosure will become apparent from the following embodiments taken in conjunction with the drawings, wherein:
- Fig. 1: shows an embodiment of a system comprising multiple trackable elements;
- Figs. 2A, 2B: show one of the trackable elements of Fig.1 in a top view and in a cross-sectional view, respectively;
- Fig. 3: shows a flow diagram of a method of operating a system comprising at least one trackable element;
- Fig. 4A: shows another trackable element in an unattached state;
- Fig. 4B: shows the trackable element of Fig. 4A attached to an object to be tracked;
- Fig. 5A: shows another trackable element in an unattached state;
- Fig. 5B: shows the trackable element of Fig. 5A attached to an object to be tracked; and
- Figs. 6A, 6B: show further trackable elements in unattached states.

### Detailed Description

In the following description of exemplary embodiments, the same reference numerals are used to denote the same or similar components.

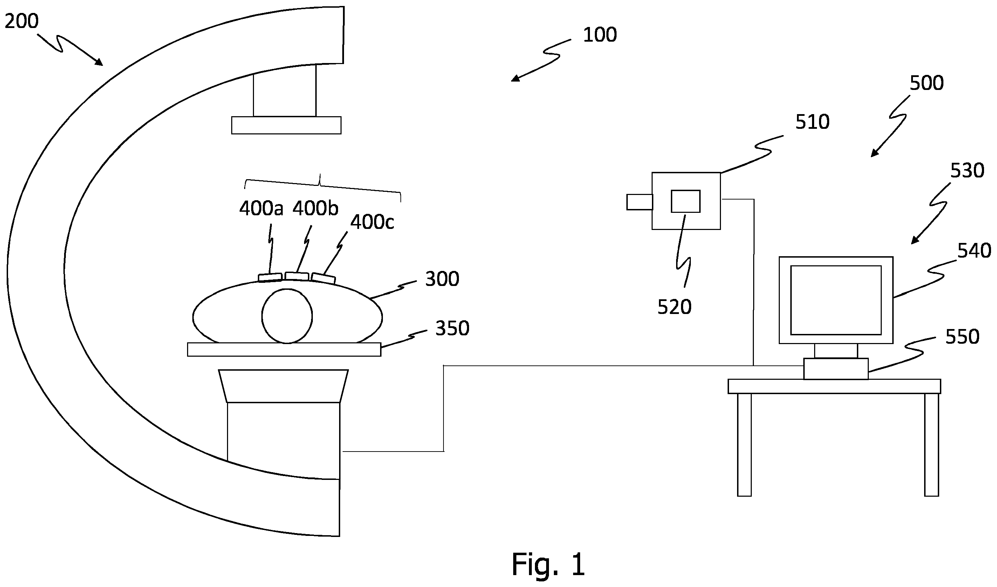

Fig. 1 shows a operating room system 100 comprising a medical imaging device 200 and a patient 300 to be imaged by the medical imaging device 200. The system 100 further comprises multiple (e.g., three or more) individual trackable elements 400a, 400b 400c collectively denoted by reference numeral 400. The trackable elements 400 are attached to the patient 300 to be located in a radiation beam path of the medical imaging device 200. As such, the trackable elements 400 may jointly be operated as patient tracker. In some surgical scenarios (e.g., spinal interventions), the trackable elements 400 may be placed around an incision.

The operating room system 100 illustrated in Fig. 1 further comprises a tracking and navigation system 500. The tracking and navigation system 500 comprises a tracking camera 510 configured to optically track the multiple trackable elements 400 and generate tracking image data representative of the trackable elements 400. The system 100 may further comprise a surgical instrument (not shown) likewise tracked by the tracking camera 510. In certain implementations, the tracking camera 510 is a stereo camera and sensitive in the infrared spectrum.

The tracking and navigation system 500 further comprises an inductive emitter 520 which, in the present embodiment, is integrated into the tracking camera 510. In other variants, the inductive emitter 520 by be attached to or integrated into a patient support 350. The inductive emitter 520 comprises at least one emitter coil (not shown).

The inductive emitter 520 is configured to generate a magnetic field for inductively transferring electromagnetic energy to the trackable elements 400. This magnetic field may be generated in synchronization with a framerate of the tracking camera 510. Such a pulsating operation of the inductive emitter 520 is beneficial in regard to reduced power consumption and reduced exposure of the patient 300 to electromagnetic radiation.

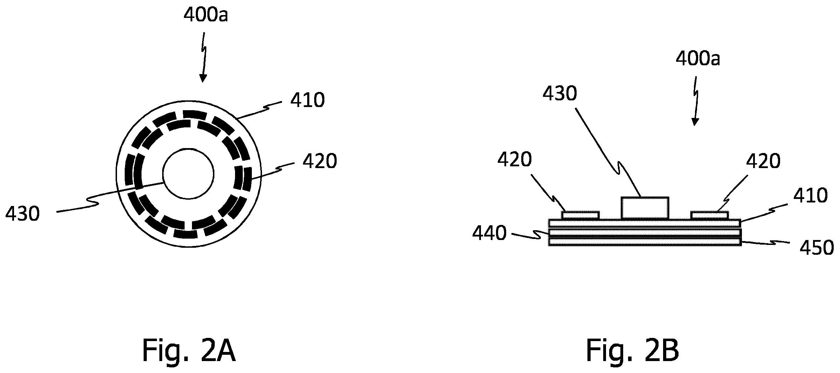

A more detailed view of a single trackable element 400a is shown in Fig. 2A (top view) and 2B (cross-sectional view). The other trackable elements 400b, 400c may be configured analogously to the trackable element 400a.

The trackable element 400a of Figs. 2A and 2B comprises a flexible substrate 410 (e.g., a flexible PCB) with an inductive receiver integrated therein. The inductive receiver comprises a receiver coil 420 configured to inductively receive energy. An adhesive layer 440, with a removable protective layer 450 arranged thereon, is provided on a side of the substrate 410 facing the patient 300. Once the protective layer 450 has been removed (e.g., peeled off), the trackable element 400a can adhesively be attached to the patient's skin like a conventional sticker, as illustrated in Fig. 1. In such and other variants, the trackable element 400a can be a disposable single-use item.

The trackable element 400a of Figs. 2A and 2B further comprises an active element 430 located on top of or within the substrate 410 and electrically coupled to the receiver coil 420 via one or more circuit lines (not shown). In the present example, the active element 430 is realized as an LED configured to emit infrared light upon the receiver coil 420 inductively receiving energy and supplying the received energy to the active element 430.

The trackable element 400a may comprise multiple electric and/or electronic components (not shown) forming a circuit for controlling the light emission of the active element 430, e.g., by setting electric parameters for a powering of the active element 430. The electric parameters may be used to control at least one of a temporal light emission pattern (e.g., continuously or non-continuously) and a frequency at which the light is emitted (e.g., for communicating with the tracking and navigation system 500). In some implementations, the trackable element 400a comprises multiple active elements 430, so that the electric and/or electronic components can individually control the active elements 430 to be on or off (e.g., so that one or more active elements 430 are on while one or more other active elements 430 are off).

The trackable element 400a may not comprise a (e.g., inductively chargeable) battery. In such a configuration, the active element 430 will substantially only be powered while its inductive energy receiver is inductively receiving energy. Lack of a battery allows the realization of a particularly light-weight trackable element 400. Moreover, production becomes more environmentally friendly and production costs are reduced.

In some variants, the trackable element 400a may comprise an (e.g., inductively chargeable) energy storage comprising at least one of a capacitor of sufficient capacity to power the active element 430 and a (e.g., re-chargeable) battery. Providing an energy storage may allow a continuous operation of the trackable element 400a, e.g., in case of an only intermittent exposure of the trackable element 400a to a field generated by the inductive energy emitter 520. The size and/or capacity of the energy storage may be chosen based on a predefined duration the trackable element 400a is to be operable without inductively receiving energy. For example, a sweet spot may be defined for the size of a capacitor or battery providing a preferable tradeoff between ensuring a continuous operation of the trackable element 400a for a predetermined duration and the weight, size and costs of the trackable element 400a. As an example, the energy storage may allow an independent operation of the active element 430 for not more than 30 seconds, not more than 1 minute, not more than 5 minutes, or not more than 10 minutes.

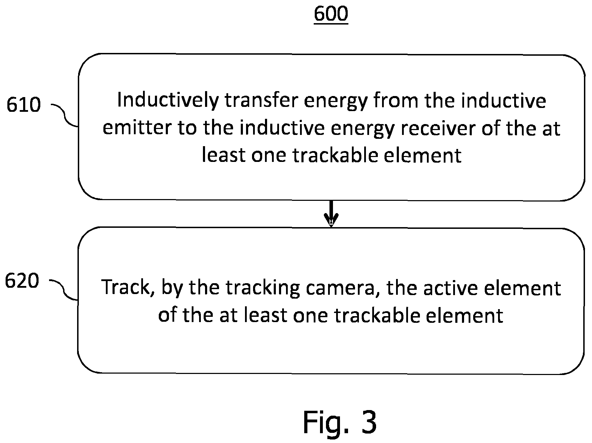

Evidently, the active elements 430 have to be electrically powered to emit light and, thus, become detectable by the tracking camera 510. According to the technique presented herein, the active elements 430 are powered using energy inductively received by the trackable element 400 according to a method as illustrated in the flow diagram 600 of Fig. 3.

In step 610, electromagnetic energy is transferred inductively from the inductive emitter 520 of Fig. 1 to the inductive receiver coils 420 of the trackable elements 400. The inductively received energy is transferred via a suitable circuit (e.g., one or more circuit lines and one or more electric components such as resistors) to the respective active element 430. As a result, the active elements 430 of the trackable elements 400 are switched on and start to emit light.

In step 620, the active elements 430 of the trackable elements 400 are tracked by the tracking camera 510. In other words, the tracking camera 510 generates tracking image data indicative of the trackable elements 400. The tracking image data may be used for one or more of image registration and navigation purposes

Referring back to Figs. 2A and 2B, at least a part of the trackable element 400a is radiopaque so that the image data generated by the medical imaging device 200 is indicative of this radiopaque part. As an example, one or more soldering spots used to electrically connect the active element 430 to a circuit line may constitute the radiopaque part of the trackable element 400a. In other variants, one or more dedicated radiopaque elements such as metal spheres may be comprised by the trackable element 400a (e.g., embedded in the substrate 410). Based on a known spatial relationship (e.g., a co-location) between the radiopaque part and the active element 430 of the trackable element 400a, medical image data generated by the medical imaging device 200 may be registered with tracking image data generated by the tracking camera 510.

In more detail, and returning to Fig. 1, the navigation system 500 of Fig. 1 comprises a computing device 530 with a display 540 and a processor 550. The computing device 530 is configured to receive the tracking image data from the tracking camera 510 and the medical image data from the medical imaging device 200 (e.g., via a cable or wirelessly). The processor 550 of the computing device 530 is configured to identify the radiopaque parts of the trackable elements 400 in the medical image data and to determine a pattern of the active elements 430 (i.e., their spatial relations and, thus, the spatial relations between the trackable elements 400 on the patient's skin) based on the identified radiopaque parts and their known spatial relationship to the respective active element 430. The processor 550 of the computing device 530 is further configured to identify the active elements 430 in the tracking image data and to determine a pattern of the active elements 430 (i.e., their spatial relations and, thus, the spatial relations between the trackable elements 400 on the patient's skin, wherein the spatial relations between the trackable elements 400 define a virtual tracker). All spatial relations may be determined in a respective coordinate system, e.g., a tracking coordinate system associated with the tracking image data and a medical coordinate system (e.g., according to the DICOM standard) associated with the medical image data.

Once the patterns of the active elements 430 and, thus, the trackable elements 400 have been determined in the respective image data, the processor 550 registers the respective coordinate systems with each other by pattern matching techniques. The computing device 530 may then use the registered image data for generating visual navigation instructions on the display 540. For example, the current position or trajectory of a surgical instrument tracked by the tracking camera 510 may be superimposed on the medical image data generated by the medical imaging device 200 (e.g., relative to a previously planned target position or target trajectory).

In some variants, the trackable elements 400 may be attached to the patient 300 in a certain pattern. The pattern may be predefined or may be determined to become known (e.g., from the tracking image data). In such or other variants, the processor 550 of the computing device 530 may be configured to determine and/or control (e.g., via visual instructions on the display 540) a placement of a further trackable element 400 to achieve a predetermined pattern. In such or other variants, the computing device 530 may be configured to determine, based on the tracking image data and based on the known pattern, a malfunction of one or the trackable element 400. Such a malfunction may occur in scenarios in which energy is transmitted by the inductive transmitter 520 but not all of the active elements 430 of the trackable elements 400 located in the field of view of the tracking camera 510 emit light. The malfunctioning trackable element 400 may be determined based on a deviation of the current pattern of trackable elements 430 from the known pattern. The malfunctioning trackable element 400 may be indicated on the display 540. It is to be reminded that the trackable elements 400 emit infrared light, so that a malfunction cannot be detected by visual inspection of the trackable elements 400.

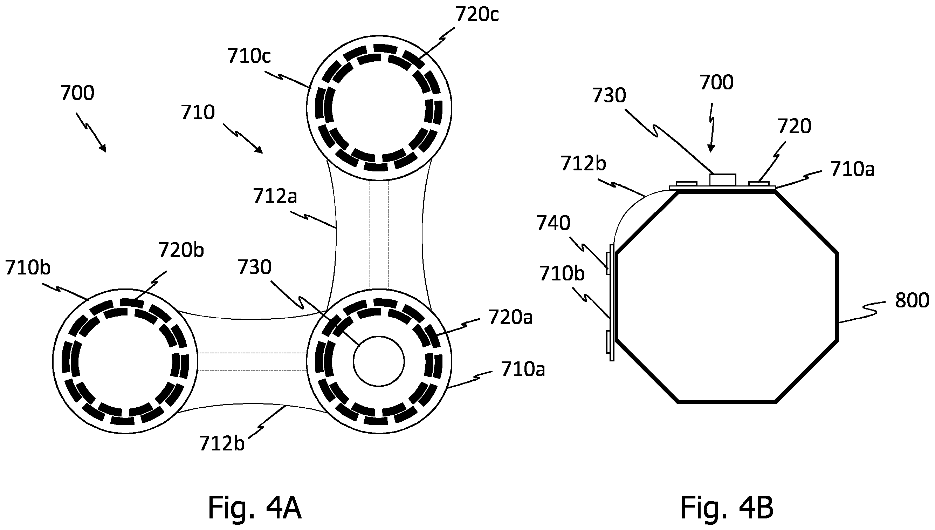

Fig. 4A shows another implementation of a trackable element 700 in an unattached configuration. The trackable element 700 comprises a substrate 710 with three (possibly flexible) receiver portions 710a, 710b, 710c interconnected by two flexible portions 712a, 712b. In other variants, the number of receiver portions 710a, 710b, 710c and/or the relative orientation of the flexible portions 712a, 712b may differ from the shown example. As an example, the receiver portion 710c and the flexible portion 712a may be omitted. As another example, one or two further receiver portions and one or more further flexible portions may be provided (e.g., to result in a T or X configuration).

In the variant shown in Fig 4A, a first receiver portion 710a of the substrate 710 is configured similarly to the trackable element 400a shown in Figs. 2A and 2B. The first receiver portion 710a comprises a first receiver coil 720a integrated into the first receiver portion 710a and an active element 730 electrically coupled to the first receiver coil 720a. The other two receiver portions 710b, 710c comprise respective second and third receiver coils 720b, 720c. Each of the receiver coils 720 is electrically coupled to the active element 730 to enable powering of the active element 730 by energy inductively received by the receiver coils 720.

Fig. 4B shows the trackable element 700 attached to an object 800. The object 800 may be a surgical instrument or an anatomical object of the patient 300 (e.g., the patient's nose) to be tracked. As such, the trackable element 700 may be operated as an instrument tracker or as a patient tracker. The flexible portions 712a, 712b allow the trackable element 700 to be (e.g., adhesively) attached to the object 800 such that the receiver portions 710a, 710b, 710c are located substantially orthogonal to each other, i.e., one receiver portion 710a on top of the object 800 (e.g., an instrument), one receiver portion 710b on the left side and one receiver portion 710c on the back side (not visible).

In some variants, the system 100 of Fig. 1 further comprises the object 800 with the trackable element 700 (and possibly further trackable elements as presented herein) attached thereto. In these variants, the tracking image data is further indicative of the trackable elements 700 attached to the object 800. Consequently, the computing device 530 is enabled to determine navigation instructions for navigating an instrument (as an exemplary object 800) relative to the medical image data based on the registered coordinate systems, as explained above.

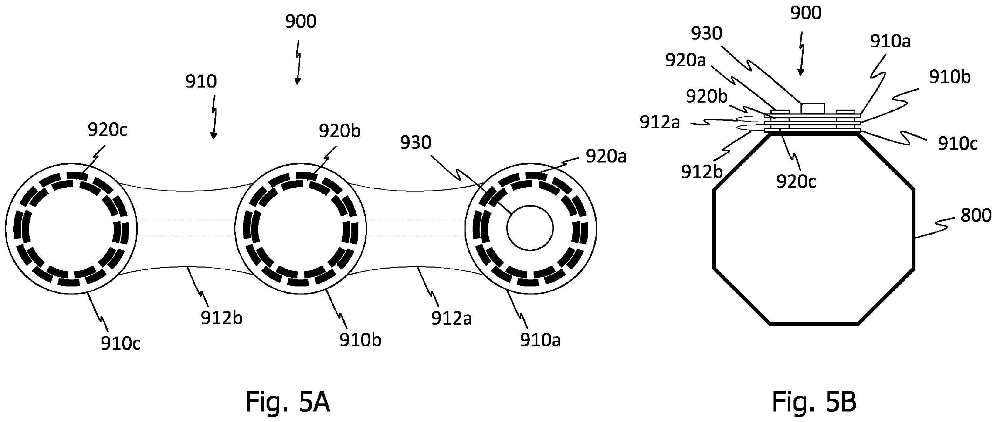

Fig. 5A shows another implementation of a trackable element 900 in an unattached configuration. Similar to the trackable element 700 shown in Fig. 4A, the trackable element 900 comprises a substrate 910 with three receiver portions 910a, 910b, 910c. The trackable element 900 shown in Fig. 5A differs from the trackable element 700 shown in Fig. 4A in that its receiver portions 910a, 910b, 910c are connected by two flexible portions 912a, 912b having the same orientation. As a result, the trackable element 900 forms a line in the unattached configuration. Further, each of the receiver portions 910a, 910b, 910c comprises a winding portion 920a, 920b, 920c of a first receiver coil 920, wherein the winding portions 920a, 920b, 920c are electrically connected in series. In this variant, the flexible portions 912a, 912b allow the receiver portions 910a, 910b, 910c to be stacked on top of each other in an attached configuration, as schematically shown in Fig. 5B. The stacked winding portions 920a, 920b, 920c of the first receiver coil 920 now form a combined coil 920. The stacking may be assisted by adhesive properties of the receiver portions 910a, 910b, 910c.

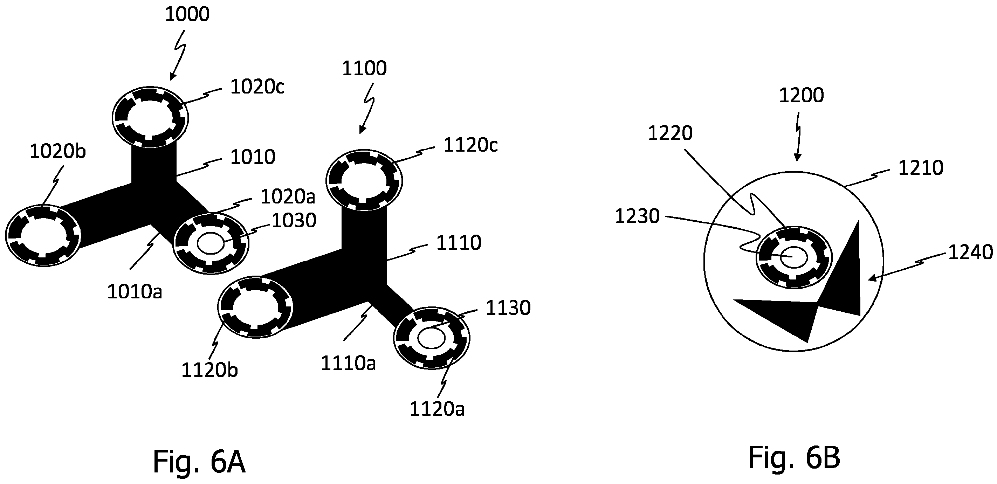

Figs. 6A and 6B show further trackable elements 1000, 1100, 1200 with respective substrates 1010, 1110, 1210, respective receiver coils 1020a-c, 1120a-c, 1220, and respective active elements 1030, 1130, 1230.

In contrast to the trackable elements 400 of Figs. 1 and 2, each of the trackable elements 1000, 1100, 1200 is optically (e.g., in tracking image data) unambiguously identifiable. The trackable elements 1000, 1100 shown in Fig 6A differ in the geometry of their substrates 1010, 1110 (e.g., in different dimensions of one or more substrate portions, such as their respective substrate arm 1010a, 1110a). The trackable element 1200 shown in Fig 6B comprises a dedicated identification element 1240 with an optical pattern (e.g., printed on the substrate 1210) defining two triangles having different orientations.

Each of the trackable elements 1000, 1100, 1200 is thus unambiguously optically identifiable in the tracking image data generated by the tracking camera 510. As a result, the computing device 530 can differentiate for example the trackable elements 1000 and 1100 in the tracking image data.

As has become apparent from the above description of exemplary implementations of the present disclosure, a trackable element with an optically detectable active element is presented that is inductively powered. As a result, the trackable element can become small and light-weight since no battery for powering the one or more active elements is needed. Consequently, there is also no need to check the battery state prior to a surgical procedure. Moreover, the shelf-live of the trackable element is increased as the shelf-live is mainly defined by the expected reliability period of the battery. Alternatively, an inductively or otherwise (re-) chargeable battery or capacitor of sufficient electrical capacity to power the active element may be provided. Such a storage for electric energy may enable bridging episodes of intermittent exposure of the trackable element to an electric field generated by an inductive energy emitter, i.e., to ensure continuous operation of the trackable element. The battery or capacitor may be comparatively small (e.g., smaller than 0,15 or 0,25 cm³) and light-weight (e.g., of not more than 15 or 25g) in comparison to batteries of conventional battery-powered trackers, since only limited time episodes without exposure to a field generated by the inductive energy emitter have to be bridged, thus still enabling a particularly small and light-weight design of the trackable element. The trackable element can be realized as a sticker that can easily be attached to the object to be tracked. Such an implementation increases the flexibility of surgical procedures.

The features described in relation to the exemplary embodiments shown in the drawings can be readily combined with further features described herein. It is apparent, therefore, that the present disclosure may be varied in many ways. Such variations are not to be regarded as a departure from the scope of the invention as defined by the claims appended hereto.

## Claims

1. A trackable element (400, 700, 900) for use in an optical tracking system, the trackable element comprising:
an active element (430, 730, 930) configured to emit light when being electrically powered; and
an inductive energy receiver comprising a first receiver coil (420, 720, 920) configured to inductively receive energy, the inductive energy receiver being configured to electrically power the active element (430, 730, 930).

2. The trackable element (400, 700, 900) according to claim 1, wherein the trackable element (400, 700, 900) includes a single active element (430, 730, 930).

3. The trackable element (400, 700, 900) according to any preceding claim, comprising:
a flexible substrate (410, 710, 910) configured to conform to a surface that is at least one of non-rigid and non-planar.

4. The trackable element (400, 700, 900) according to any preceding claim, comprising
a printed circuit board, PCB, configured to electrically couple the inductive energy receiver and the active element (430, 730, 930).

5. The trackable element (400, 700) according to claims 3 and 4, wherein
the PCB is a flexible PCB that constitutes the flexible substrate (410, 710).

6. The trackable element (400, 700, 900) of claim 4 or 5, wherein
at least the first receiver coil (420, 720, 920) is integrated into the PCB.

7. The trackable element (700, 900) according to any preceding claim, wherein
the inductive energy receiver comprises a second receiver coil (720b, 920b) configured to inductively receive energy, wherein the second receiver coil (720b, 920b) is, or is configured to be, substantially orthogonally oriented relative to the first receiver coil (720a, 920a), and, optionally, wherein
the inductive energy receiver comprises a third receiver coil (720c, 920c) configured to inductively receive energy, wherein the third receiver coil is, or is configured to be, substantially orthogonally oriented relative to the first and the second receiver coils (720a, 720b; 920a, 920b).

8. The trackable element (700, 900) according to claim 7, wherein
at least one of the second receiver coil (720b, 920b), the third receiver coil (720c, 920c) and at least one further receiver coil is integrated into the PCB.

9. The trackable element (400) according to any preceding claim, comprising:
an attachment element (440) configured for adhesively attaching the trackable element (400) to an object (800) that is to be tracked.

10. The trackable element (400, 700, 900) according to any preceding claim, comprising at least one of:
components configured to set electric parameters for a powering of the active element; and
components configured to wireless transfer data.

11. The trackable element (400, 700, 900, 1200) according to any preceding claim, wherein at least a part of the trackable element (400, 700, 900, 1200) is radiopaque; and/or
wherein i) the active element (430, 730, 930) is shaped in an optically unambiguous geometric form or ii) the trackable element comprises another active or passive optically detectable element (1240) shaped in an optically unambiguous geometric form.

12. The trackable element (900) according to any preceding claim, further comprising
at least one flexible portion (912a, 912b), and wherein the first coil comprises at least two separate winding portions (920a, 920b, 920c) connected by the at least one flexible portion (912a, 912b), the at least one flexible portion (912a, 912b) configured to place the at least two winding portions (920a, 920b, 920c) on top of each other.

13. A system (100) comprising:
at least one trackable element (400, 700, 900) according to any of the preceding claims; and
an inductive energy emitter (520) configured to inductively transfer energy to the inductive energy receiver of the at least one trackable element (400, 700, 900).

14. The system (100) according to claim 13, wherein
the inductive energy emitter (520) comprises one or more emitter coils.

15. The system (100) according to any of claims 13 or 14, comprising:
a tracking camera (510) configured to track the at least one trackable element (400, 700, 900).

16. The system (100) according to claim 15, wherein
the tracking camera (510) has a framerate, when wherein the inductive emitter (520) is configured to generate a magnetic field in synchronization with the framerate of the tracking camera (510); and/or wherein
the inductive emitter (520) is integrated into the tracking camera (510).

17. The system (100) according to any of claims 13 to 16, wherein
the system (100) comprises multiple trackable elements (400, 700, 900) according to any of claims 1 to 12; and wherein
the inductive emitter (520) is configured to simultaneously transfer energy to the inductive receivers of at least two of the multiple trackable elements (700, 900).

18. A method (600) of operating a system (100) comprising at least one trackable element (400, 700, 900) according to any of claims 1 to 12, an inductive energy emitter (520) configured to inductively transfer energy to the inductive energy receiver of the at least one trackable element (400, 700, 900), and a tracking camera (510) configured to track the at least one trackable element (400, 700, 900), the method comprising:
inductively transferring (610) energy from the inductive emitter (520) to the inductive energy receiver of the at least one trackable element (400, 700, 900); and
tracking (620), by the tracking camera (510), the active element (430, 730, 930) of the at least one trackable element (400, 700, 900).

19. The method (600) according to claim 18, wherein at least two trackable elements (400, 700, 900) are attached to an object (800), and further comprising:
tracking the at least two trackable elements (400, 700, 900) attached to the object (800) by the tracking camera (510); and
attaching at least one further trackable element (400, 700, 900) based on tracking image data generated by the tracking camera (510) and indicative of the at least two trackable elements (400, 700, 900) attached to the object (800), in particular to realize a target pattern of trackable elements (700, 900) on the object (800).

20. The method (600) according to claim 18, wherein multiple trackable elements (400, 700, 900) are attached to the object (800) in a user-selected pattern, and further comprising:
determining the user-selected pattern based on tracking image data generated by the tracking camera (510); and at least one of
i) determining at least one of a position and an orientation of the object (800) based on the determined user-selected pattern;
ii) determining a malfunction of any of the multiple trackable elements (400, 700, 900) based on the determined user-selected pattern.

## Amended claims

### Amended claims in accordance with Rule 137(2) EPC.

1. A trackable element for use in an optical tracking system, the trackable element comprising:
an active element configured to emit light when being electrically powered; and
an inductive energy receiver comprising a first receiver coil configured to inductively receive energy and a second receiver coil configured to inductively receive energy, wherein the second receiver coil is orthogonally aligned to the first receiver coil, the inductive energy receiver being configured to electrically power the active element.

2. The trackable element according to claim 1, wherein
the trackable element includes a single active element.

3. The trackable element according to any preceding claim, comprising:
a flexible substrate configured to conform to a surface that is at least one of non-rigid and non-planar.

4. The trackable element according to any preceding claim, comprising
a printed circuit board, PCB, configured to electrically couple the inductive energy receiver and the active element.

5. The trackable element according to claims 3 and 4, wherein
the PCB is a flexible PCB that constitutes the flexible substrate.

6. The trackable element of claim 4 or 5, wherein
at least the first receiver coil is integrated in the PCB.

7. The trackable element according to any preceding claim, wherein
the inductive energy receiver comprises a third receiver coil configured to inductively receive energy, wherein the third receiver coil is orthogonally aligned to the first and the second receiver coils.

8. The trackable element according to claim 7, wherein at least one of the second receiver coil, the third receiver coil and at least one further receiver coil is integrated in the PCB.

9. The trackable element according to any preceding claim, comprising:
an attachment element configured for adhesively attachment of the trackable element to an object that is to be tracked.

10. The trackable element according to any preceding claim, comprising:
electronic components configured to set electric parameters for a powering of the active element; and/or
electronic components configured to wireless transfer data.

11. The trackable element according to any preceding claim, wherein at least a part of the trackable element is radiopaque; and/or
wherein i) the active element is shaped in an optically unambiguous geometric form or ii) the trackable element comprises another active or passive optically detectable element shaped in an optically unambiguous geometric form.

12. The trackable element according to any preceding claim, further comprising at least one flexible portion and wherein the first coil comprises at least two separate winding portions connected by the at least one flexible portion, the at least one flexible portion allowing to place the at least two winding portions on top of each other.

13. A system comprising:
at least one trackable element according to any of the preceding claims; and
an inductive energy emitter configured to inductively transfer energy to the inductive energy receiver.

14. The system according to claim 13, wherein
the inductive energy emitter comprises one or more emitter coils.

15. The system according to any of claims 13 or 14, comprising:
a tracking camera configured to track the at least one trackable element.

16. The system according to claim 15, wherein
the inductive emitter is configured to generate a magnetic field in synchronization with a framerate of the tracking camera; and/or wherein
the inductive emitter is integrated into the tracking camera.

17. The system according to any of claims 13 to 16, wherein
the system comprises multiple trackable elements according to any of claims 1 to 12, and wherein the inductive emitter is configured to simultaneously transfer energy to the inductive receivers of at least two of the multiple trackable elements.

18. A method of operating a system comprising at least one trackable element according to any of claims 1 to 12, an inductive energy emitter configured to inductively transfer energy to the inductive energy receiver of the at least one trackable element, and a tracking camera configured to track the at least one trackable element, the method comprising:
inductively transferring energy from the inductive emitter to the inductive energy receiver of the at least the trackable element; and
tracking, by the tracking camera, the active element of the at least the trackable element.

19. The method according to claim 18, wherein at least two trackable elements are attached to an object, and further comprising:
tracking the at least two trackable elements attached to the object; and
attaching at least one further trackable element based on tracking data indicative of the at least two trackable elements attached to the object to achieve a selected pattern of trackable elements on the object.

20. The method according to claim 18, wherein multiple trackable elements are attached to the object in a user-selected pattern, and further comprising:
determining the selected pattern based on the tracking data; and
determining a pose of the object based on the determined selected pattern.
